# EUROPEAN PATENT APPLICATION

(11) **EP 3 812 451 A1**
(43) Date of publication of application: **28.04.2021**
(21) Application number: 19840469.1
(22) Date of filing: 12.06.2019
(51) Int. Cl.: C12M 1/42, C12N 5/07

(54) **CELL STIMULATION DEVICE, CELL STIMULATION METHOD, PRODUCTION METHOD FOR CULTURE PRODUCT, PRODUCTION METHOD FOR ISOLATED CELLS, AND CELL PROLIFERATION METHOD**

(30) Priority: 25.07.2018 JP 2018139593
(71) Applicant: Able Corporation, Tokyo 162-0812 (JP)
(72) Inventor: KOYAMA, Sumihiro, Tokyo 162-0812 (JP); TAMURA, Yasuyuki, Tokyo 162-0812 (JP); ISHIKAWA, Yoichi, Tokyo 162-0812 (JP)
(74) Representative: Pitchford, James Edward
(86) International application number: PCT/JP2019/023345
(87) International publication number: WO 2020/021896

(57) **Abstract**

A cell stimulating apparatus for detaching adhesive cells adhered on a culture substrate from the culture substrate by stimulating the cells, comprising: application means for applying an alternating field between a first electrode and a second electrode opposing each other by sandwiching cells and a solution in which the cells are dipped, wherein at least one of the first electrode and the second electrode is not in contact with the cells and the solution.

## Description

### TECHNICAL FIELD

The present invention relates to a cell stimulating apparatus, a cell stimulating method, a culture product manufacturing method, an isolated cell manufacturing method, and a cell growing method.

### BACKGROUND ART

PTL1 discloses a technique for detaching cells attached on an electrode by using a three-electrode culture system.

### CITATION LIST

### PATENT LITERATURE

PTL1: Japanese Patent No. 5515094

### SUMMARY OF INVENTION

### TECHNICAL PROBLEM

In this technique described in PTL1, however, the arrangement becomes complicated because the three-electrode culture system is used and cells must be cultured on electrodes.

The present invention has been made in consideration of the above problem, and has as its object to provide a technique capable of easily detaching cells with a simple arrangement.

### SOLUTION TO PROBLEM

To achieve the above object, a cell stimulating apparatus according to the present invention is a cell stimulating apparatus for detaching adhesive cells adhered on a culture substrate from the culture substrate by stimulating the cells, characterized by including
application means for applying an alternating field between a first electrode and a second electrode opposing each other by sandwiching cells and a solution in which the cells are dipped,
wherein at least one of the first electrode and the second electrode is not in contact with the cells and the solution.

### ADVANTAGEOUS EFFECTS OF INVENTION

The present invention can easily detach cells with a simple configuration.

Other features and advantages of the present invention will be apparent from the following description taken in conjunction with the accompanying drawings. Note that the same reference numerals denote the same or like components throughout the accompanying drawings.

### BRIEF DESCRIPTION OF DRAWINGS

The accompanying drawings, which are incorporated in and constitute a part of the specification, illustrate embodiments of the invention and, together with the description, serve to explain principles of the invention.
Fig. 1 is a view showing the frequency characteristic of the dielectric loss of water;
Fig. 2 is a view showing a configuration example of a cell stimulating apparatus according to an embodiment of the present invention;
Fig. 3A is a view showing the way CHO-K1 cells were detached with time;
Fig. 3B is a view showing the measurement results of the cell detachment of CHO-K1 cells;
Fig. 4A is a view showing the measurement results of the cell detachment and the viability of CHO-K1 cells as functions of the field strength;
Fig. 4B is a view showing the measurement results of the cell detachment and the viability of CHO-K1 cells as functions of the frequency;
Fig. 5 is a view showing the comparison results of growth curves after cell subculture was performed by an alternating field and a trypsin treatment;
Fig. 6A is a view showing the way BALB/3T3 cells were detached with time;
Fig. 6B is a view showing the measurement results of the cell detachment and the viability of BALB/3T3 cells as functions of the field strength;
Fig. 6C is a view showing the measurement results of the cell detachment and the viability of BALB/3T3 cells as functions of the frequency;
Fig. 7A is a view showing the way CHO-K1 cells in stacked flasks were detached with time, when an alternating field was applied to the cells vertically;
Fig. 7B is a view showing the way CHO-K1 cells in stacked flasks were detached with time, when an alternating field was applied to the cells sideways (horizontally);
Fig. 7C is a view showing the measurement results of the cell detachment and the viability under different conditions;
Fig. 8A is a view showing the way cells were detached with time in PBS(+) containing calcium;
Fig. 8B is a view showing the measurement results of the cell detachment and the viability under different conditions;
Fig. 9 is a view showing a configuration example of a cell stimulating apparatus according to an embodiment of the present invention;
Fig. 10A is a view showing the way CHO-K1 cells on laminin-coated electrodes were detached with time;
Fig. 10B is a view showing the cell detachment of the CHO-K1 cells on the laminin-coated electrodes;
Fig. 10C is a view showing the viability of the CHO-K1 cells on the laminin-coated electrodes;
Fig. 11A is a view showing the way CHO-K1 cells on a vitronectin-coated culture flask were detached with time;
Fig. 11B is a view showing the way CHO-K1 cells on a vitronectin-coated culture flask were detached with time;
Fig. 11C is a view showing the measurement results of the cell detachment and the viability under different conditions;
Fig. 12A is a view showing the way iPS cells were detached when no alternating field was applied;
Fig. 12B is a view showing the way iPS cells were detached when an alternating field was applied;
Fig. 13A is a view showing the way cells were grown with time in control for comparison;
Fig. 13B is a view showing the way cells were grown when an alternate field was applied;
Fig. 13C is a view showing the measurement results of the cell density corresponding to the elapse of time in the control and when the alternate current was applied;
Fig. 14 is a view showing changes in value of the cell density with time when an alternate current was applied and was not applied;
Fig. 15A is a view showing cells grown on microcarrier beads;
Fig. 15B is a view showing the measurement results of the number of cells under various conditions;
Fig. 15C is a view showing the measurement results of the viability under the various conditions;
Fig. 16 is a view showing a configuration example of a cell stimulating apparatus according to an embodiment of the present invention;
Fig. 17A is a view showing the way cells were detached with time;
Fig. 17B is a view showing the way cells were detached with time; and
Fig. 17C is a view showing the results of the cell detachment and the viability.

### DESCRIPTION OF EMBODIMENTS

Embodiments will be explained below with reference to the accompanying drawings. Note that arrangements to be disclosed in the following embodiments are merely examples, so the present invention is not limited to these arrangements.

A cell stimulating apparatus according to an embodiment of the present invention is configured as an apparatus that changes the behavior and properties of cells by stimulating the cells. The outline of the cell stimulating apparatus will be described below.

### (Types of Cells)

Cells to which the cell stimulating apparatus can give stimulation are not limited, so the apparatus is applicable to various cells. Examples are cells of animals, insects, and plants, microorganisms, and bacteria. Examples of animal-derived cells are cells of a human, a monkey, a dog, a cat, a rabbit, a rat, a nude mouse, a mouse, a guinea pig, a pig, sheep, a Chinese hamster, a cow, a marmoset, and an African green monkey, but they are not particularly limited. The apparatus is also applicable to various stem cells (for example, iPS cells, ES cells, mesenchymal stem cells, and adipose-derived stem cells), established cells, and tumor cells.

### (States of Cells)

The states of cells to which the cell stimulating apparatus gives stimulation are not particularly limited.

For example, the cell stimulating apparatus can be so configured as to stimulate cells adhered on a culture substrate. Examples of the culture substrate are a flat substrate, a spherical or granular substrate called a microcarrier, and a cylindrical substrate called a roller bottle. The flat culture substrate can also be the culture surface of a member (a so-called culture flask, petri dish, or stack plate) that is generally used for culture. As the flat culture substrate, it is also possible to apply a flat metal plate, or a conductive member on the glass surface of which an ITO electrode is formed. Note that various processes (an etching process for adjusting the surface roughness, and a coating process) for culturing cells may also be performed on the surface of the culture substrate.

The cell stimulating apparatus can also be configured to stimulate cells in a state in which they are isolated from a culture substrate, cells in a state in which they form an aggregate, cells in a state of tissues, cells in a state of living bodies, cells in a state of seeds, and cells in a state of sprouted plants.

The cell stimulating apparatus is so configured as to stimulate cells in a state in which they are dipped in a solution (including a state in which cells are dipped in a body fluid in the form of tissues or living bodies, and a state in which cells are extracted from a living body and dipped in a solution). For example, cells can undergo a stimulating process in a state in which they are held in a vessel such as a culture flask or a petri dish together with a solution. The type of the solution is not particularly limited, and it is possible to apply various solutions in accordance with the types of cells and the purposes of stimulation. For example, a culture solution such as a serum medium or PBS(-) not containing calcium and magnesium can be used as the solution. Furthermore, a liquid containing an additive matching the purpose can be used as the solution.

### (Purposes)

The cell stimulating apparatus stimulates cells for the purpose of changing the behavior or properties of the cells, and there are various examples of the purpose. For example, the cell stimulating apparatus can be used for various purposes such as detachment of cells from a culture substrate (including detachment in the form of a sheet or the like, and detachment in the form of a separated single cell), a form change, growth suppression, gene expression, protein generation, degeneration of a culture product, a change in generation amount, differentiation induction, and initialization. The cell stimulating apparatus can also be used for the purposes of curing living things (animals including a human and plants), and controlling growth.

### (Configuration)

The cell stimulating apparatus includes an application means capable of applying an alternating field between two electrodes, and stimulates (applies the generated alternating field (an alternating electric field) to) cells placed in the alternating field. Examples of the waveform of the alternating field are a sine wave, a square wave, a triangular wave, and a sawtooth wave, but the waveform is not limited to these examples. The sine wave can be used as an example. Cells are placed in the alternating field as they are dipped in a solution, and at least one of the two electrodes is not brought into contact with the cells and the solution. Note that the two electrodes may also be so-called parallel plate electrodes obtained by arranging plate-like electrodes parallel to each other. In this case, the layout of the electrodes is not particularly limited, and they can be laid out to sandwich cells and a solution vertically or sideways (horizontally). In addition, when stimulating cells contained in a vessel, the electrodes can be so configured as to sandwich one vessel or a plurality of vessels.

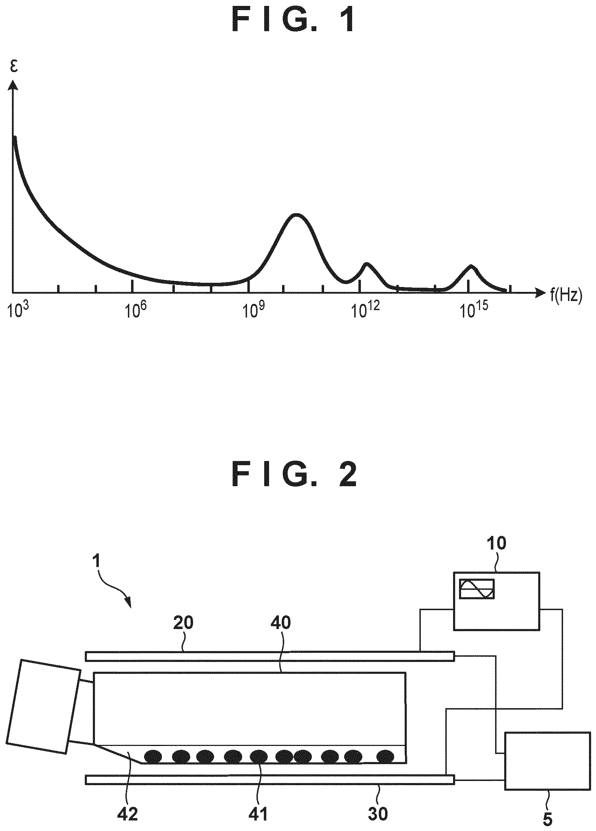

As the frequency of the alternating field to be generated by the cell stimulating apparatus, a range within which the dielectric loss of water has a predetermined value or less can be applied. For example, as shown in Fig. 1 showing the frequency characteristic of a dielectric loss ε of water, an alternating field having a frequency of 0.5 to 1,000 MHz decreases the dielectric loss ε of water, so an alternating field in this range can be used.

The strength (field strength) of the alternating field to be generated by the cell stimulating apparatus can be determined in accordance with the purpose of cell stimulation, and it was confirmed that even an alternating field of about ±0.3 V/m changed the behavior of cells by stimulating them (to be described in detail later).

The cell stimulating apparatus according to the embodiment of the present invention will be explained below by taking more practical examples.

### [First Embodiment]

### <Configuration of Cell Stimulating Apparatus>

A configuration example of a cell stimulating apparatus according to an embodiment of the present invention will be explained below with reference to Fig. 2. A cell stimulating apparatus 1 for stimulating cells includes a function generator 10. The function generator 10 functions as an application means capable of applying an alternating field between an electrode 20 (a first electrode) and an electrode 30 (a second electrode). The function generator 10 applies an alternating field to stimulate cells arranged between the electrode 20, and the electrode 30 opposing the electrode 20. The cell stimulating apparatus 1 also includes a voltmeter 5 (an oscilloscope). The voltmeter 5 can measure the voltage between the two electrodes 20 and 30. An alternating field (electric field) can be calculated based on the measured voltage, and the distance between the two electrodes 20 and 30. However, the cell stimulating apparatus can also be configured so as not to include any voltmeter. That is, a field having a desired density can be applied between the electrodes by confirming the set value of the function generator 10 and the alternating field to be applied between the electrodes in advance, and adjusting the set value of the function generator 10.

The function generator 10 is an electronic device capable of generating an alternating voltage signal having a desired frequency and a desired waveform. When the function generator 10 is connected between the electrodes 20 and 30, an alternating field can be generated between the electrodes 20 and 30. Note that the type of the function generator 10 applicable to this embodiment is not particularly limited, but it is preferable to select and design the function generator 10 having outputs (a frequency, a voltage, and a waveform) corresponding to target cells and the purpose of stimulation.

The types of the electrodes 20 and 30 are not particularly limited either, and it is possible to use, for example, transparent electrodes (indium tin oxide (ITO)/glass electrodes) and metal electrodes such as stainless steel, copper, and aluminum. In addition, the electrodes 20 and 30 can be formed as, for example, plate electrodes. When plate electrodes are arranged parallel to face each other, it is possible to generate an electric field uniformly (with no unevenness) between the electrodes. Note that the plate electrode includes not only a so-called plate-like form but also a practically plate-like form. Examples are a plate electrode having a mesh structure, an electrode having end portions bent inward in order to avoid dispersion of an electric field in the peripheral portions, and an electrode entirely moderately curved in order to obtain a uniform electric field between electrodes.

In this embodiment, the electrodes 20 and 30 are so arranged as not to come in contact with cells to be stimulated and a solution in which the cells are dipped. For example, it is possible to encapsulate cells and a solution in a vessel, and place this vessel between the electrodes 20 and 30. That is, the electrodes 20 and 30 can be placed outside the vessel. Since the electrodes are not in contact with the cells and the solution, it is possible to prevent the physical properties of the electrodes 20 and 30 from influencing the cells. Note that when the electrodes 20 and 30 are placed outside the vessel, the vessel is preferably made of a material having small influence on the field. Since the field generated between the electrodes 20 and 30 is not easily influenced by the vessel, the field can uniformly act on the cells. A practical example is a resin vessel not containing metals and magnetic substances. In addition, when the electrodes 20 and 30 are placed outside the vessel, a cell stimulating process can be performed without opening and closing the vessel. This makes it possible to reduce the risk of the occurrence of contamination in the vessel. Furthermore, it is unnecessary to specially devise the vessel, the cell stimulating process can be performed by using a generally circulated culture vessel.

In this embodiment, a culture flask 40 is used as the vessel, and the function generator 10 applies an alternating field in a state in which the culture flask 40 is placed between the two electrodes 20 and 30.

The culture flask 40 contains cells 41 and a liquid 42, and the cells 41 between the two electrodes 20 and 30 are stimulated by using the function generator 10. The inner surface (the inner wall surface of the bottom) of the culture flask 40 is a culture substrate, and the cells 41 can be adhesive cells adhered on the inner surface of the culture flask 40. Alternatively, the cells 41 can be placed in the culture flask 40 in a state in which the cells 41 adhere to fine spherical or granular culture substrates called microcarriers.

The frequency of the alternating field to be applied between the electrodes can appropriately be adjusted in accordance with the conditions such as the type of cells and the purpose of stimulation. In one embodiment, the frequency of the alternating field is 0.1 MHz or more, preferably 0.5 MHz or more, more preferably 2 MHz or more, and further preferably 5 MHz or more, and is 1,000 MHz or less, preferably 200 MHz or less, more preferably 50 MHz or less, and further preferably 25 MHz or less. The efficiency of cell stimulation can be improved by thus selecting the frequency in accordance with the purpose of cell stimulation.

Also, in one embodiment, the frequency (band) of the alternating field to be applied between the electrodes can be set based on the dielectric loss of water. That is, when an alternating electric field is applied to water, a dielectric loss occurs because ions in the water move at a low frequency, and the dielectric loss decreases as the frequency rises. When using an alternating field (an alternating field of about 0.5 (inclusive) to 1,000 (inclusive) MHz) in a region in which the dielectric loss of water decreases, the movement of ions in the solution is minimized. Since stimulation by the electric field, not stimulation caused by the movement of ions, can directly be given to cells, the cell stimulation efficiency can improve.

The strength (field strength) of the alternating field to be applied between the electrodes is not particularly limited, and can properly be set in accordance with the type of cells and the purpose of stimulation. For example, the strength of the alternating field can be ±0.3 V/m or more.

In one embodiment, the strength of the alternating field is ±1 mV/2.6 cm or more, preferably ±5 mV/2.6 cm or more, more preferably ±15 mV/2.6 cm or more, and particularly preferably ±50 mV/2.6 cm or more, and is ±10,000 mV/2.6 cm or less, preferably ±3,000 mV/2.6 cm or less, and more preferably ±2,000 mV/2.6 cm or less. If the distance between the electrodes is longer than or shorter than 2.6 cm, the voltage need only be increased or decreased in proportion to the distance. The cell stimulation efficiency can be improved by thus selecting the field strength in accordance with the purpose of cell stimulation.

Note that practical values of the frequency and field strength of the alternating field to be applied to cells are suitably set in accordance with the type of cells and the purpose of stimulation, and optimum values can experimentally be derived.

In one embodiment, the strength (field strength) of the alternating field, the application time of the alternating field, and the environment in which cells are placed when the alternating field is applied are set appropriately in accordance with the type of cells and the purpose of stimulation, and their optimum values can experimentally be derived.

In one embodiment, the cell stimulating apparatus 1 can include a measuring means for measuring the voltage between the electrodes 20 and 30. In this case, the output of the function generator 10 can be adjusted based on the measurement value of the measuring means. Consequently, a field (electric field) having a desired strength can be applied between the electrodes 20 and 30.

The vessel appliable to this embodiment is not particularly limited either. A culture flask has been taken as an example of the vessel, but any known vessel such as a petri dish can also be used. Also, it is possible to use only one vessel as shown in Fig. 2, but the present invention is not limited to this. That is, it is possible to stack a plurality of vessels vertically, or align a plurality of vessels horizontally.

The present invention can stimulate cells placed between the two electrodes by only applying an alternating field between the two electrodes by using the function generator, and hence can stimulate cells with a simple arrangement.

### <Cell Stimulating Method>

Next, a cell stimulating method according to an embodiment of the present invention will be explained. This cell stimulating method includes a step of preparing cells to be stimulated, as preparations. Cells can be prepared by culture, but it is also possible to perform a step of obtaining cultured cells and stimulating the cells. Although cells adhering to a culture substrate can be used, the present invention is not limited to this. For example, it is possible to apply isolated cells or an aggregate of cells. Cells can also be a tissue slice, and the tissue slice can be in the state of a living body or in a state separated from a living body. Cells are prepared as they are dipped in a liquid. For example, cells can be prepared in a state in which they are contained in a vessel together with a liquid. This liquid can properly be selected in accordance with the purpose of stimulation. The liquid can be a culture solution used in culture, and can also be a liquid (a solution suitable for a cell stimulating step) obtained by substituting the culture solution.

The cell stimulating method includes a step of placing the cells 41 and the liquid 42 in which the cells 41 are dipped, between the electrodes 20 and 30. When the cells 41 and the liquid 42 are contained in the culture flask 40, this step can be implemented by placing the culture flask 40 between the electrodes 20 and 30. Note that this step is performed such that at least one of the electrodes 20 and 30 is not in contact with the cells 41 and the liquid 42. In the example shown in Fig. 2, both the electrodes 20 and 30 are not in contact with the cells 41 and the liquid 42.

The cell stimulating method includes a step of applying an alternating field between the electrodes 20 and 30. The alternating field (the frequency, waveform, strength, and time) to be applied in this step can appropriately be set in accordance with the purpose of stimulation. It is also possible to appropriately set the environment (the temperature, humidity, illumination, and brightness) of cells in this step in accordance with the purpose of stimulation.

The above steps make it possible to stimulate cells and manufacture various products. For example, when using the cell stimulating method for the purpose of detaching (isolating) cells from a culture substrate, isolated cells detached (isolated) from the culture substrate can be obtained as a product. The present invention is also applicable to a cell growing method of culturing and growing isolated cells detached from a culture substrate by cell stimulation. In addition, when using the cell stimulating method in order to adjust the physical property change or the production amount of a metabolite of cells, this metabolite of cells can be obtained as a product. Furthermore, when using the stimulating method for degeneration (for example, gene expression, differentiation induction, or initialization) of cells, degenerated cells can be obtained as a product.

### (Example 1)

In Example 1, detachment of cells on a culture substrate will be explained as an example of stimulation to cells. Note that the detachment as the ratio of detachment of cells is calculated by measuring the cell density at random in six portions on a 500-µm × 500-µm culture substrate before and after the detaching process. If the detachment exceeds 95%, it is determined that detachment is complete. The viability as the ratio at which detached cells are viable is calculated by using a viability test by Trypan blue dyeing.

### <Contents and Results of Experiments>

The contents of experiments on cell detachment conducted by using an alternating field will be explained. Before the experiments, the cells 41 were cultured to a subconfluent of 80% or more in the T-25 culture flask 40. After the culture, the culture solution was removed, and the cells 41 in the T-25 culture flask 40 were lightly washed with the liquid 42 (a phosphate buffer (PBS(-)) not containing calcium and magnesium). After the washing, the liquid was replaced with 5 mL of a fresh liquid 42 (PBS(-)). After the addition of 5 mL of PBS(-), the depth was about 2 mm.

The T-25 culture flask 40 had a thickness of about 2.6 cm and a volume of about 50 mL, and the portion above PBS(-) in the flask was filled with air. The upper and lower portions of the T-25 culture flask 40 were sandwiched between the two electrodes 20 and 30, and a ±80-mV/2.6 cm, 20-MHz alternating field was applied between the two electrodes at room temperature. After the alternating field was applied for 10 min, pipetting was performed 7 to 10 times by using the 5-mL PBS(-) in the T-25 culture flask 40, and the presence/absence of cell stimulation was checked.

### [CHO-K1 Cell]

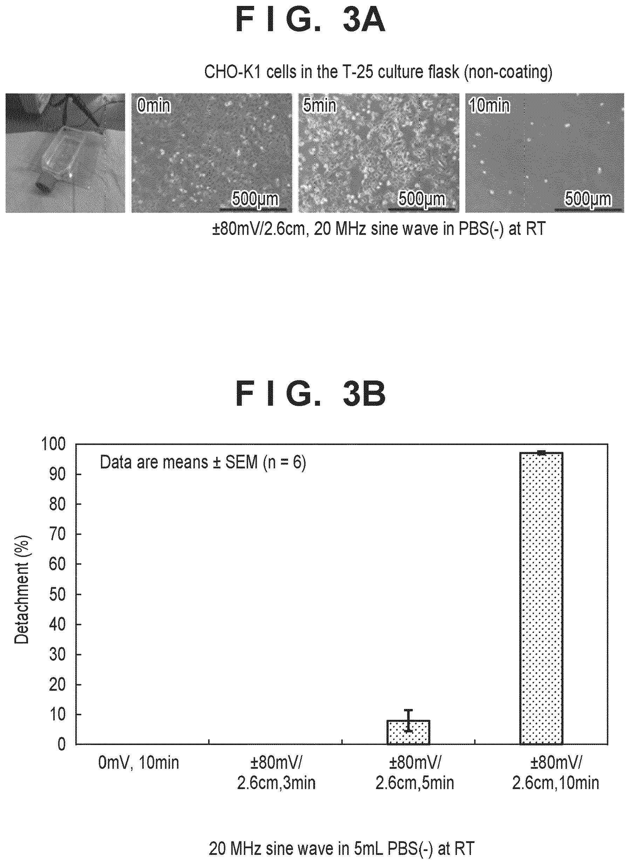

Consequently, as shown in Figs. 3A and 3B, when CHO-K1 cells were used as the cells 41, 5-min alternating field application performed by the function generator 10 detached 10% of the cells attached on the culture substrate, and 10-min alternating field application completely detached the cells.

More specifically, Fig. 3A shows the CHO-K1 cells in PBS(-) when the CHO-K1 cells were stimulated for 0 min, 5 min, and 10 min by applying the ±80-mV/2.6 cm, 20-MHz alternating field. Fig. 3B shows the detachment of the CHO-K1 cells. The leftmost result in Fig. 3B shows the result obtained for 0 mV and 10 min, that is, obtained when the CHO-K1 cells were left to stand in PBS(-) at room temperature for 10 min without applying any alternating field. In this case, no CHO-K1 cells were detached from the culture substrate surface. The second result from the left shows the result obtained when the ±80-mV/2.6 cm, 20-MHz alternating field was applied for 3 min, and almost no CHO-K1 cells were detached from the culture substrate surface in this case as well. The third result from the left shows the result obtained when the ±80-mV/2.6 cm, 20-MHz alternating field was applied for 5 min, and the detachment was less than 10% in this case. The rightmost result shows the result obtained when the ±80-mV/2.6 cm, 20-MHz alternating field was applied for 10 min, and the detachment exceeded 95%. That is, complete detachment occurred.

It was thus confirmed that when the alternating field was applied, cells attached on the culture substrate were stimulated and detached.

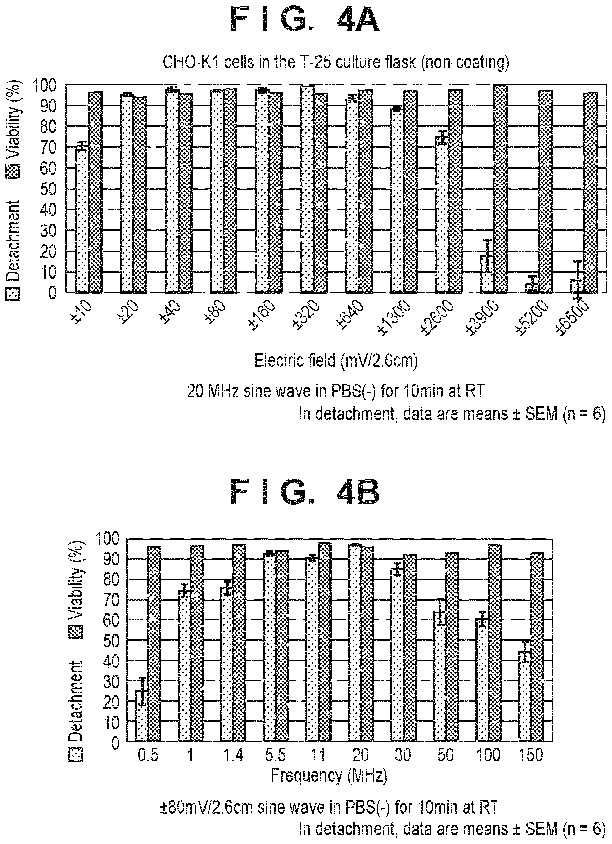

Fig. 4A shows the measurement results of the cell detachment and the viability of detached cells when 20-MHz alternating fields within the range of ±10 to ±6,500 mV/2.6 cm were applied to CHO-K1 cells in PBS(-) for 10 min. Referring to Fig. 4A, bars on the left side indicate the cell detachment, and bars on the right side indicate the viability, for the individual application conditions.

It was confirmed from Fig. 4A that cells were detached within a broad range of field strengths. In particular, it was confirmed that the cell detachment reached 90% within the range of ±20 to ±640 mV/2.6 cm. It was also confirmed that cells were detached even by a low field strength of ±10 mV/2.6 cm, and cell detachment occurred even when the field strength exceeded ±1,300 mV/2.6 cm although the cell detachment decreased. Furthermore, it was confirmed that a high viability exceeding 80% was obtained by field strengths within a broad range of ±10 to ±6,500 mV/2.6 cm.

That is, it was confirmed that cells received stimulation and changed the behavior when an electric field of ±10 mV/2.6 cm was applied. In particular, it was confirmed that cells were detached by applying electric fields of ±10 to ±6,500 mV/2.6 cm, and the cell detachment increased when electric fields of ±10 to ±2,600 mV/2.6 cm were applied.

Fig. 4B shows the measurement results of the cell detachment and the viability of detached cells when ±80 mV/2.6 cm alternating fields within the range of 0.5 to 150 MHz were applied to CHO-K1 cells in PBS(-) for 10 min. Referring to Fig. 4B, bars on the left side indicate the cell detachment, and bars on the right side indicate the viability, for the individual application conditions.

That is, it was confirmed that cells received stimulation and changed the behavior when an electric field having a frequency of 0.5 MHz or more was applied. In particular, it was confirmed that cells were detached by applying alternating fields of 0.5 to 150 MHz, and the cell detachment increased when alternating fields of 1 to 100 MHz were applied.

It was confirmed from Fig. 4B that cells were detached within a broad range of frequencies. In particular, it was confirmed that the detachment exceeded 90% within the range of 5.5 to 20 MHz. It was also confirmed that when the frequency was lower or higher than this range, the cell detachment decreased, but cells were still detached. Furthermore, it was confirmed that a high viability exceeding 90% was obtained within a broad range of frequencies.

Figs. 4A and 4B particularly reveal that the viability exceeded 90% for 20-MHz alternating fields within the range of ±10 to ±6,500 mV/2.6 cm, and for ±80-mV/2.6 cm alternating fields within the range of 0.5 to 150 MHz.

In addition, a growth curve which cells subcultured by the application of an alternating field showed after that was examined in comparison with a growth curve when a trypsin treatment was performed. More specifically, a ±80-mV/2.6 cm, 20-MHz alternating field was applied vertically to the T-25 culture flask 40 substituted with PBS(-) at room temperature for 10 min, thereby detaching CHO-K1 cells. Also, after washing was performed with PBS(-), a treatment was performed by 1 × trypsin EDTA (an aqueous solution prepared by diluting a 10 × trypsin EDTA solution 10 times with PBS(-)) at 37°C for 10 min, thereby detaching the CHO-K1 cells from the T-25 culture flask 40. The detached cells were seeded at a cell density of 1 × 10⁵ cells/T-25 culture flask. Then, the cell density was measured by setting 0 hour when 3 hours elapsed after the cell seeding.

The growth of animal cells generally shows a growth curve including four phases, that is, an induction phase (or a lag phase, lag time), a log phase, a stationary phase, and a death phase. The induction phase is a period during which cells do not divide and become adaptive to a new environment. The length of the induction phase depends on the cell density and the time of damage recovery after seeding.

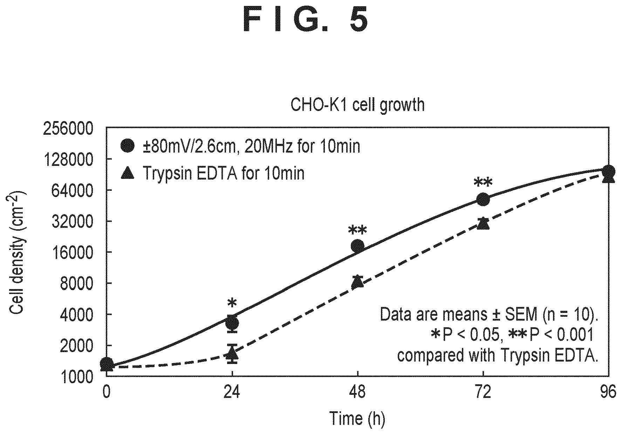

Fig. 5 is a view showing the results of comparison between a growth curve obtained by the application of an alternating field, and a growth curve after cell subculture was performed by a trypsin treatment, when the abovementioned examination was performed. As shown in Fig. 5, unlike the growth curve obtained when the trypsin treatment was performed, the growth curve of CHO-K1 cells obtained by the alternating field had almost no induction phase, and immediately entered the log phase. That is, as shown in Fig. 5, cells detached by the application of the alternating field grew after that, and the lag time (induction phase) of the growth was shorter than that when the trypsin treatment was performed. Especially in a period of 0 to 24 hours, the rise of the cell density when the alternating field was applied was larger than that when the trypsin treatment was performed.

### [BALB/3T3 Cell]

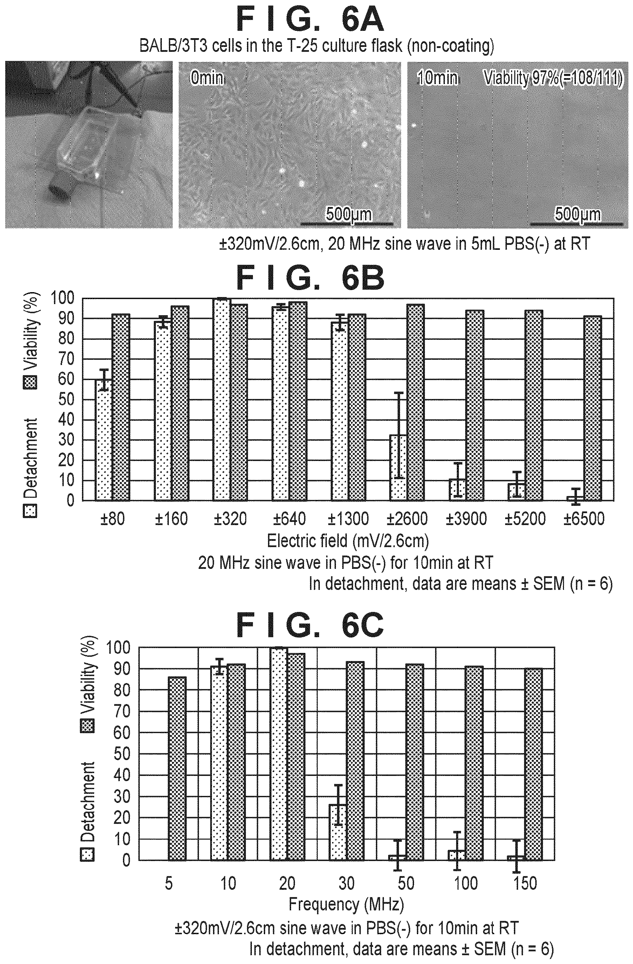

The results obtained when using BALB/3T3 cells as the cells 41 will be explained below. Fig. 6A shows BALB/3T3 cells in PBS(-) when the BALB/3T3 cells were stimulated by applying a ±320 mV/2.6 cm, 20-MHz alternating field for 10 min. This 10-min alternating field application completely detached the cells.

Fig. 6B shows the measurement results of the cell detachment and the viability of detached cells when 20-MHz alternating fields within the range of ±80 to ±6,500 mV/2.6 cm were applied to BALB/3T3 cells in PBS(-) for 10 min. In Figs. 6B and 6C, bars on the left side indicate the cell detachment, and bars on the right side indicate the viability, for the individual application conditions.

It was confirmed from Fig. 6B that the cells were detached by field strengths in a broad range. In particular, it was confirmed that the cell detachment reached 80% within the range of ±160 to ±1,300 mV/2.6 cm. It was also confirmed that even when the field strength became lower or higher than this range, the cells were detached although the cell detachment decreased. Furthermore, it was confirmed that a high viability exceeding 90% was obtained for field strengths within a broad range of ±80 to ±6,500 mV/2.6 cm.

Fig. 6C shows the measurement results of the cell detachment and the viability of detached cells when ±320-mV/2.6 cm alternating fields within the range of 0.5 to 150 MHz were applied to BALB/3T3 cells in PBS(-) for 10 min.

It was confirmed from Fig. 6C that the detachment exceeded 90% within the range of 10 to 20 MHz. When the frequency was lower or higher than this range, the cell detachment tended to decrease. It was also confirmed that a high viability exceeding 80% was held within a broad range of frequencies.

In particular, Figs. 6B and 6C reveal that when the application conditions were 20 MHz and ±320 to ±640 mV/2.6 cm, complete detachment occurred, and the viability exceeded 90%. Also, the viability exceeded 90% when the application conditions were 20 MHz and ±80 to ±6,500 mV/2.6 cm. In addition, the viability exceeded 90% when the application conditions were ±320 mV/2.6 cm and 10 to 100 MHz.

As described above, it is possible to obtain the effect of stimulating and detaching various kinds of cells by the application of an alternating field. To detach cells, field strengths and frequencies within broad ranges are applicable. By selecting particularly effective field strengths and frequencies, the cell detachment can be improved while holding the cell viability. Also, it was found that a field strength and a frequency suitable for improving the cell detachment change in accordance with the type of cells. Even when using cells other than the cells used in the example, a field strength and a frequency to be applied can be selected by referring to the abovementioned results.

### [Second Embodiment]

In the second embodiment, as an example of stimulation to cells, a plurality of T-25 culture flasks 40 in which CHO-K1 cells are cultured as cells 41 are prepared and stacked in the vertical direction, and an alternating field is applied vertically or sideways. The processing efficiency can improve because a plurality of vessels (for example, the T-25 culture flasks) can be processed at once by using the same apparatus configuration as shown in Fig. 2.

Note that vessels need not be stacked in the vertical direction, and a plurality of vessels may also be arranged in the horizontal direction. When a plurality of vessels are arranged, the distance between electrodes increases, but a desired field strength can be held by adjusting the voltage in accordance with the distance.

### (Example 2)

As in Example 1, the cells 41 (CHO-K1 cells) were cultured to a subconfluent of 80% or more in the T-25 culture flask 40 in advance. After the culture, the culture solution was removed, and the cells 41 in the T-25 culture flask 40 were lightly washed with a liquid 42 (PBS(-)). After the washing, the liquid 42 was replaced with a 5-mL fresh liquid 42 (PBS(-)). Two T-25 culture flasks 40 were stacked and sandwiched between electrodes 20 and 30, and a 62-mVpp/cm (= ±31-mV/cm = ±80-mV/2.6 cm), 20-MHz alternating field was applied between the two electrodes at room temperature. After the alternating field was applied for 10 min, pipetting was performed 7 to 10 times by using the 5-mL PBS(-) in the T-25 culture flask 40, and the presence/absence of cell detachment was checked.

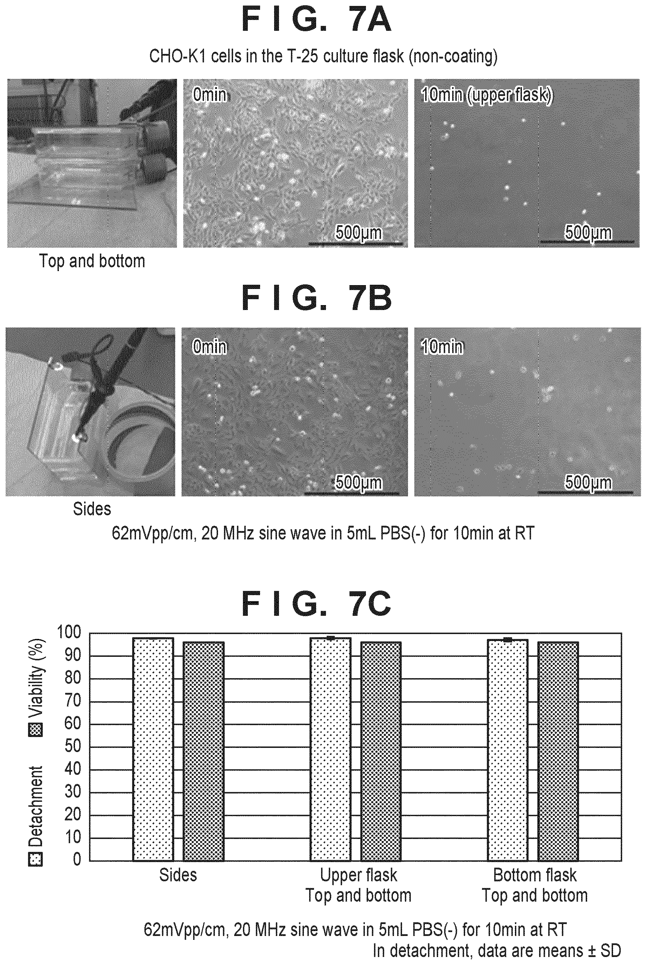

Consequently, the result obtained when the two T-25 culture flasks 40 were stacked, the two electrodes 20 and 30 were arranged above and below the stack, and the alternating field was applied vertically, as shown by "Top and bottom" in Fig. 7A, was almost the same as the result obtained when the electrodes 20 and 30 were arranged on the side surfaces of the T-25 culture flasks 40 and the alternating field was applied sideways, as shown by "Sides" in Fig. 7B. That is, the CHO-K1 cells were completely detached at a viability of 96% regardless of the application conditions. Thus, cell detachment can be performed independently of the application direction of the alternating field, and smooth cell detachment can be implemented even when a plurality of T-25 culture flasks 40 exist.

As described above, even when a plurality of vessels containing cells to be processed exist, it is possible to obtain the effect of stimulating and detaching the cells by the application of an alternating field. Also, even when using cells other than the cells used in the example, a field strength and a frequency to be applied can be selected by referring to the abovementioned results.

### [Third Embodiment]

The result of cell stimulation changes in accordance with the type and amount of a solution 42. This will be explained in the third embodiment.

### (Example 3)

As in Example 1, cells 41 (CHO-K1 cells) were cultured to a subconfluent of 80% or more in a T-25 culture flask 40 in advance.

After the culture, the culture solution was removed, and the cells 41 in the T-25 culture flask 40 were lightly washed with the liquid 42 (PBS(-)). After the washing, the liquid 42 was replaced with a 5-mL fresh liquid 42 (PBS(-)). Two T-25 culture flasks 40 were sandwiched between electrodes 20 and 30, and a 62-mVpp/cm (= ±31-mV/cm = ±80-mV/2.6 cm), 20-MHz alternating field was applied between the two electrodes at room temperature. After the alternating field was applied for 10 min, pipetting was performed 7 to 10 times by using the 5-mL PBS(-) in the T-25 culture flask 40, and the presence/absence of cell detachment was checked (condition 1).

For comparison, the presence/absence of cell detachment was confirmed under the same condition as condition 1 except that the liquid 42 was replaced with a 1-mL fresh liquid 42 (PBS(-)) after the washing (condition 2). For comparison, the presence/absence of cell detachment was checked under the same condition as condition 1 except that the liquid 42 (PBS(-)) was removed (0 mL) after the washing (condition 3). For comparison, the presence/absence of cell detachment was checked under the same condition as condition 1 except that no alternating field was applied (condition 4).

For comparison, the presence/absence of cell detachment was checked under the same condition as condition 1 except that the washing was performed by using calcium-containing PBS(+) and the liquid was replaced with 5-mL fresh PBS(+) after the washing (condition 5). For comparison, the presence/absence of cell detachment was checked under the same condition as condition 1 except that the culture medium (1 mL) used in the culture was directly used and no washing was performed (condition 6).

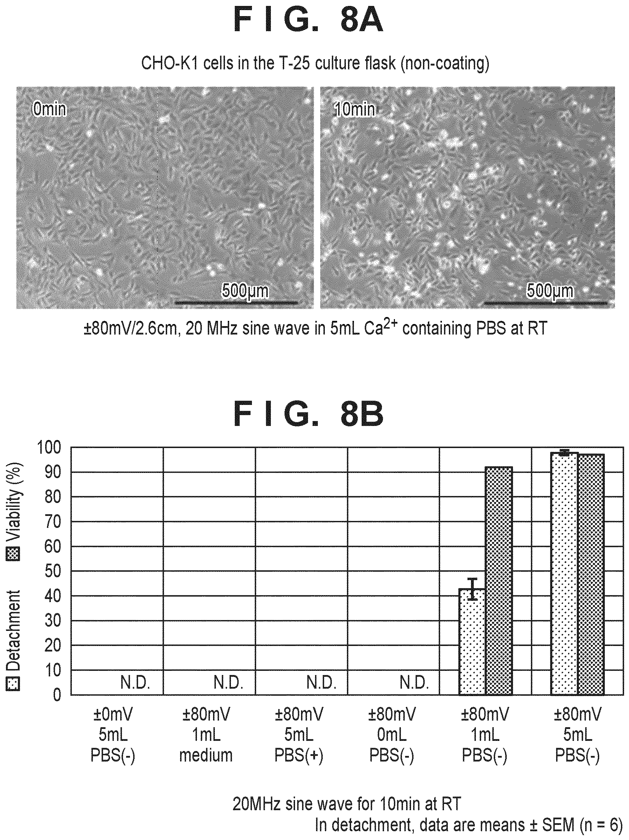

Fig. 8A shows CHO-K1 cells in calcium-containing PBS(+) when the CHO-K1 cells were stimulated for 0 min and 10 min by applying a ±80-mV/2.6 cm, 20-MHz alternating field at room temperature. The cells were not detached even after the elapse of 10 min.

Fig. 8B shows the measurement results of the cell detachment and the viability under the individual conditions. N.D. in the viability shown in Fig. 8B represents that no data was measured.

No CHO-K1 cells were detached when no alternating field was applied (the leftmost data (condition 4) in Fig. 8B), and when the ±80-mV/2.6 cm, 20-MHz alternating field was vertically applied for 10 min (the second data (condition 6) from the left in Fig. 8B).

Also, no detachment occurred even when the ±80-mV/2.6 cm, 20-MHz alternating field was vertically applied for 10 min to the CHO-K1 cells in PBS(+) to which 0.9-mM calcium chloride was added (the third data (condition 5) from the left in Fig. 8B). Note that Fig. 8A corresponds to the third example from the left in Fig. 8B.

When the CHO-K1 cells were washed with PBS(-), PBS(-) was removed (0 mL), and the ±80-mV/2.6 cm, 20-MHz alternating field was vertically applied for 10 min, no detachment occurred (the third data (condition 3) from the right in Fig. 8B) even when pipetting was performed by adding PBS(-) after that.

After CHO-K1 cells were washed with PBS(-), the PBS(-) was removed, and 1 mL of PBS(-) was added again. As a consequence, the depth was almost 0.4 mm. When a ±80-mV/2.6 cm, 20-MHz alternating field was vertically applied for 10 min, the detachment was 42.7% (the second data (condition 2) from the right in Fig. 8B). Note that the rightmost data (condition 1) in Fig. 8B was the same as the fifth data from the right in Fig. 4B, that is, complete detachment occurred, and the viability exceeding 95% was obtained.

As described above, CHO-K1 cells in a culture medium and in PBS(+) were not detached even when an alternating field was applied. Also, when the amount of PBS(-) was reduced (for example, from 5 mL to 1 mL), the cell detachment also decreased when an alternating field was applied.

From the foregoing, it was found that when cells are stimulated, the behavior of the cells changes in accordance with the type and amount of a solution.

### [Fourth Embodiment]

In the fourth embodiment, cell stimulation in a system in which one electrode is in contact with cells and a solution in which the cells are dipped will be explained.

### (Example 4)

### <Configuration of Cell Detaching Apparatus>

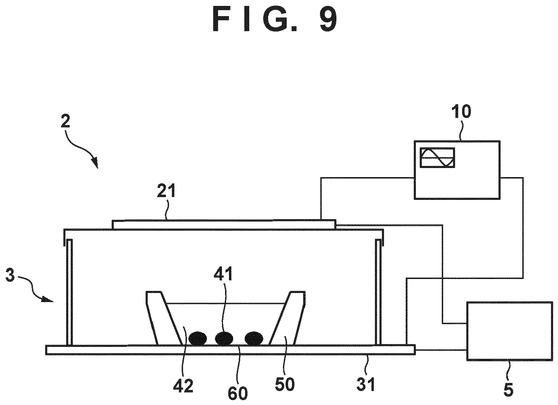

A configuration example of a cell stimulating apparatus according to an embodiment of the present invention will be explained with reference to Fig. 9. A cell stimulating apparatus 2 includes a function generator 10, and the function generator 10 functions as an application means for applying an alternating field to cells 41 (CHO-K1 cells) in a culture petri dish 3 formed by two electrodes 21 and 31. The electrode 21 is an ITO/PET film electrode or the like, and the electrode 31 is an ITO/glass electrode or the like.

### <Contents and Results of Experiments>

As shown in Fig. 9, the culture petri dish 3 to which an alternating field can be applied was formed by arranging the electrode 21 (an ITO/PET film electrode) in the upper portion, and the electrode 31 (an ITO/glass electrode) in the bottom portion. First, flexiPERM 50 was attached on the electrode 31 (an ITO/glass electrode), and a 5-µg/cm² laminin coat 60 was formed. The cells 41 (CHO-K1 cells) were cultured to a subconfluent of 80% or more on the laminin-coated electrode 31 (an ITO/glass electrode). Then, the culture medium was replaced with a liquid 42 (PBS(-)), and ±80-mV/1.1 cm alternating fields having various frequencies were applied at room temperature for 10 min.

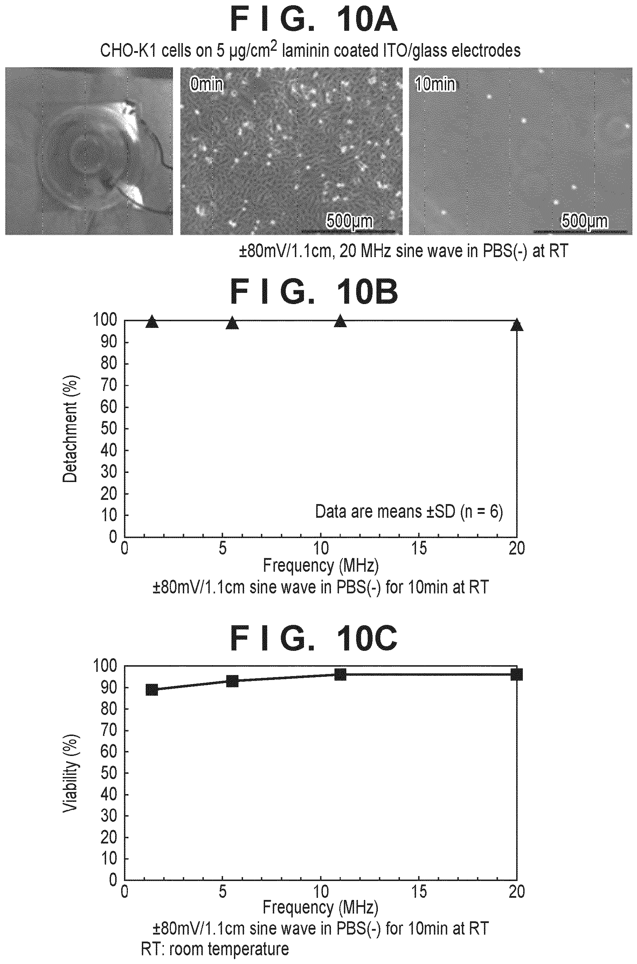

Fig. 10A shows the CHO-K1 cells when the CHO-K1 cells were stimulated for 0 min and 10 min under the abovementioned conditions. Fig. 10B shows the measurement result of the cell detachment under the above conditions, and Fig. 10C shows the measurement result of the viability under the above conditions.

As shown in Fig. 10B, the CHO-K1 cells were completely detached when the application conditions were 1.4, 5.5, 11, and 20 MHz. Note that Fig. 10A corresponds to the result when the application condition was 20 MHz shown in Fig. 10B, and a state in which the cells were detached can be observed.

Also, as shown in Fig. 10C, the CHO-K1 cells had a viability exceeding 90% for the 5.5-, 11-, and 20-MHz alternating fields.

As described above, even when one of the counter electrodes is in contact with cells and a solution, it was confirmed that the cells were stimulated within a broad range of frequencies, and it was possible to obtain a high cell detachment and a high viability.

### [Fifth Embodiment]

In the fifth embodiment, the influence of a coating agent on the cell detachment and the viability will be explained. Note that the configuration of a cell stimulating apparatus is the same as that explained with reference to Fig. 2, so an explanation thereof will be omitted.

### (Example 5)

### <Contents and Results of Experiments>

Cells 41 (CHO-K1 cells) were cultured in a T-25 culture flask 40 coated with 0,5-µg/cm² vitronectin, and cell detachment was observed when an alternating field was applied in a liquid 42 (PBS(-)) at room temperature for 10 min.

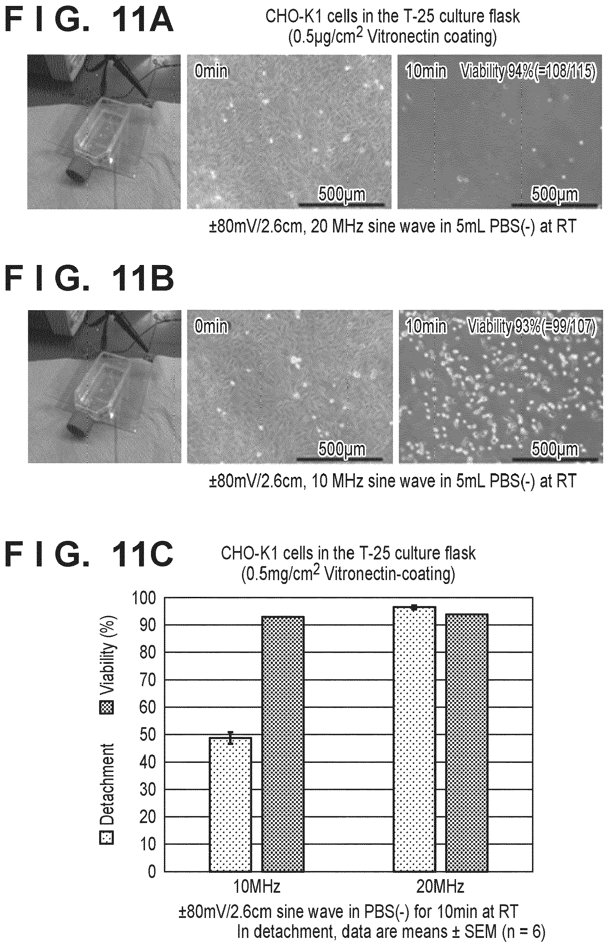

Fig. 11A shows the result when a ±80-mV/2.6 cm, 20-MHz alternating field was applied (room temperature, 10 min). Fig. 11B shows the result when a ±80-mV/2.6 cm, 10-MHz alternating field was applied (room temperature, 10 min).

Fig. 11C shows the measurement results of the cell detachment and the viability when a ±80-mV/2.6 cm alternating field was applied at room temperature for 10 min. As shown in this graph, complete detachment occurred when the 20-MHz alternating field was applied, but the detachment was lower than 50% when the 10-MHz alternating field was applied. On the other hand, when no coating material was used as shown in Fig. 4B in the above-described embodiment, the detachment was about 90% when the alternating fields having 5.5 and 11 MHz comparatively close to 10 MHz were applied. This reveals that the cell detachments were different when the (uncoated) culture flask was used as a culture substrate, and when the vitronectin-coated surface was used as a culture substrate. That is, it was confirmed that the difference between the culture substrates had influence on cells.

### [Sixth Embodiment]

In the sixth embodiment, cell stimulation will be explained by taking detachment of iPS cells as an example. Consequently, it was found that iPS cells are stimulated as well. An apparatus configuration is the same as that shown in Fig. 2, so an explanation thereof will be omitted.

### (Example 6)

### <Contents and Results of Experiments>

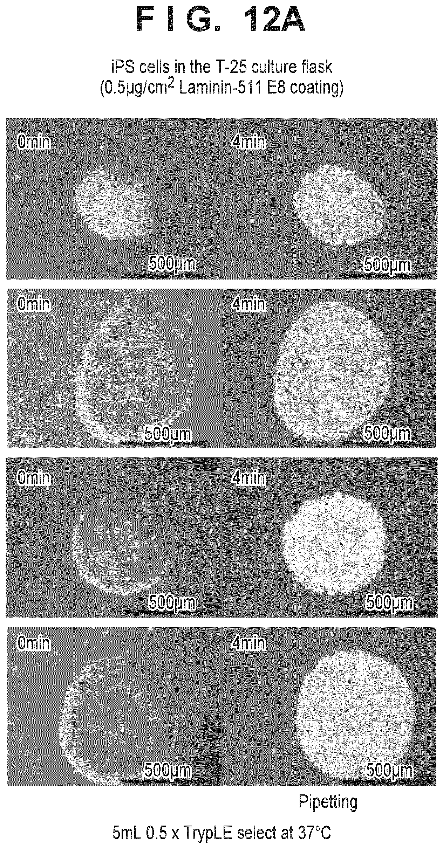

Cells 41 (iPS cells (771-2 strain, passage number = 6)) were cultured in a T-25 culture flask 40 coated with 0,5-µg/cm² laminin 511 E8. After the iPS cells were washed with a liquid 42 (PBS(-)), 5 mL of 0.5 × TrypLE select were added. The iPS cells were placed in a 5%-CO₂ incubator, and a TrypLE select treatment was performed at 37°C for 4 min. After that, pipetting was performed 10 times by TrypLE select. Fig. 12A shows the results. As shown in Fig. 12A, the colony of the iPS cells was hardly detached from the culture substrate surface.

Then, when performing the TrypLE select treatment for 4 min, a ±320-mV/2.6 cm, 20-MHz alternating field was applied to iPS cells prepared in the same manner as described above. After the 4-min alternating field application and the TrypLE select treatment, tapping as a detaching method weaker than pipetting was performed 20 times from the sides of the T-25 culture flask 40.

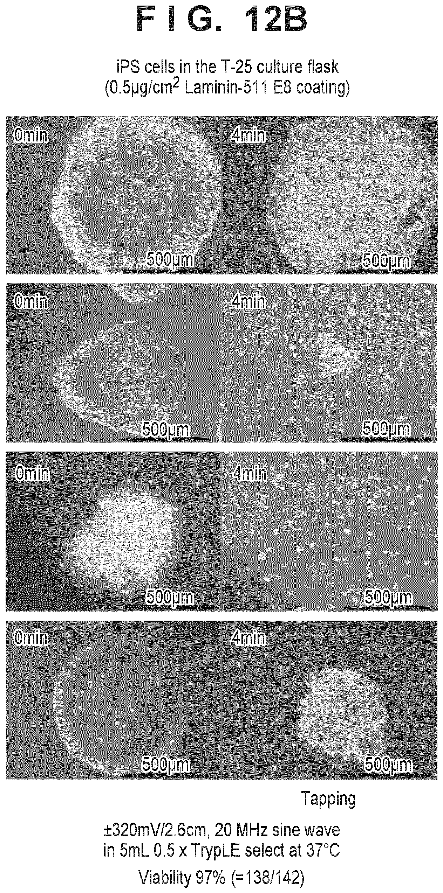

Fig. 12B shows the results. The colony of the iPS cells was entirely or partially detached. Also, the viability of the detached iPS cells was 97%. It was thus confirmed that the application of the alternating field facilitated stimulation and detachment of cells even when the cells were iPS cells.

### [Seventh Embodiment]

In the seventh embodiment, an example in which the application of an alternating field suppresses (inhibits) growth and an example in which the growth normally resumes when the application of the alternating field is stopped will be explained as examples of stimulation to cells. In this embodiment, CHO-K1 cells in a Ham's F-12 culture medium were cultured while a ±0.5-V/cm, 100-MHz alternating field was applied over 2 days, and whether the growth rate was influenced was checked. Consequently, it was found that the cells were stimulated and the growth rate was suppressed. It was also confirmed that the CHO-K1 cells restarted growing normally when the application of the ±0.5-V/cm, 100-MHz alternating field was stopped after the growth of the cells was suppressed. As a consequence, it was found that the cells started growing normally again. An apparatus configuration is the same as that shown in Fig. 2, so an explanation thereof will be omitted.

### (Example 7)

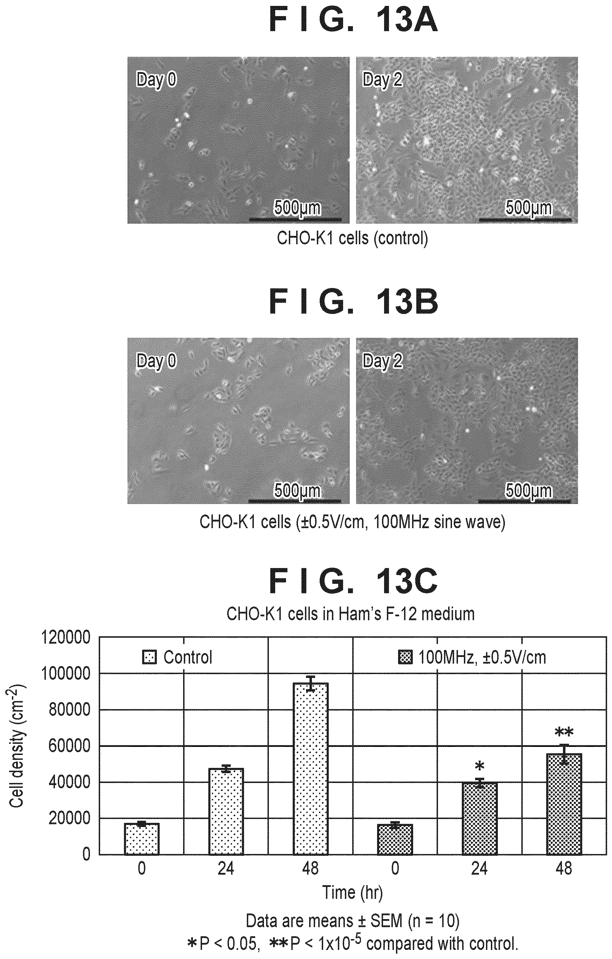

Fig. 13A is a view showing the way CHO-K1 cells grew in control for comparison. Fig. 13B is a view showing the way CHO-K1 cells grew when a ±0.5-V/cm, 100-MHz alternating field was applied to the CHO-K1 cells.

Fig. 13C shows the measurement results of the cell density under the conditions shown in Figs. 13A and 13B. Each of 0, 24, and 48 on the abscissa represents the time from the start of growth, and each value on the ordinate represents the cell density. Three bars on the left side correspond to Fig. 13A, and indicate the results in the control. Three bars on the right side correspond to Fig. 13B, and indicate the results when the ±0.5-V/cm, 100-MHz alternating field was applied.

Consequently, when the alternating field was applied, statistically significant CHO-K1 cell growth suppression was confirmed after the culture was performed for 24 hours, and the cell density became 60% of the control in 48 hours.

It was thus confirmed that the application of the ±0.5-V/cm, 100-MHz alternating field suppressed the growth of CHO-K1 cells. Accordingly, the cell stimulating apparatus according to this embodiment was found to function as a cell growth suppressing apparatus.

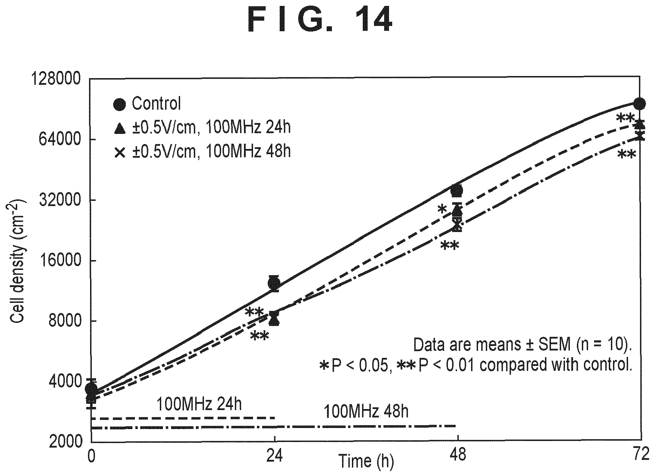

Fig. 14 is a view showing changes (the states of growth) in value of the cell density (cells/cm²) with time. Fig. 14 shows the results of three cases, that is, when no ±0.5-V/cm, 100-MHz alternating field was applied (control), when the application was performed for 24 hours and then stopped, and when the application was performed for 48 hours and then stopped.

At the time of 24 hours, the cell density was 66% of that of the control for comparison, so the growth was suppressed. That is, the cell density increased by a gradient lower than that of the control.

When the application of the alternating field was stopped after the elapse of 24 hours, the cell density increased by the same gradient as that of the control between 24 and 48 hours. At the time of 48 hours, the growth advanced so that the cell density was 81% of that of the control.

On the other hand, when the application of the alternating field was stopped after the application was performed for 48 hours, the cell density increased by a gradient lower than that of the control from 0 to 48 hours. After the elapse of 48 hours, the gradient became steep, and the cell density increased by the same gradient as that of the control.

It was thus found that when the application of the alternating field was stopped, the cells started normally growing again. Also, after the application of the alternating field was stopped, the gradient of the growth became the same as that of the control. Accordingly, it was confirmed that the growth was suppressed not because the cells were damaged by the application of the alternating field but because the growing function was simply suppressed while the alternating field was applied.

### [Eighth Embodiment]

In the eighth embodiment, as an example of stimulation to cells, an alternating field was applied by a function generator to CHO-K1 cells cultured on microcarrier beads. Consequently, it was found that even the cells cultured on the microcarrier beads were stimulated and detached by the application of the alternating field. Note that an apparatus configuration is the same as that of Fig. 2, so an explanation thereof will be omitted. Note also that polystyrene beads manufactured by CORNING are used as the microcarrier beads, but the microcarrier beads were not limited to the polystyrene beads and may also be other microcarrier beads.

### (Example 8)

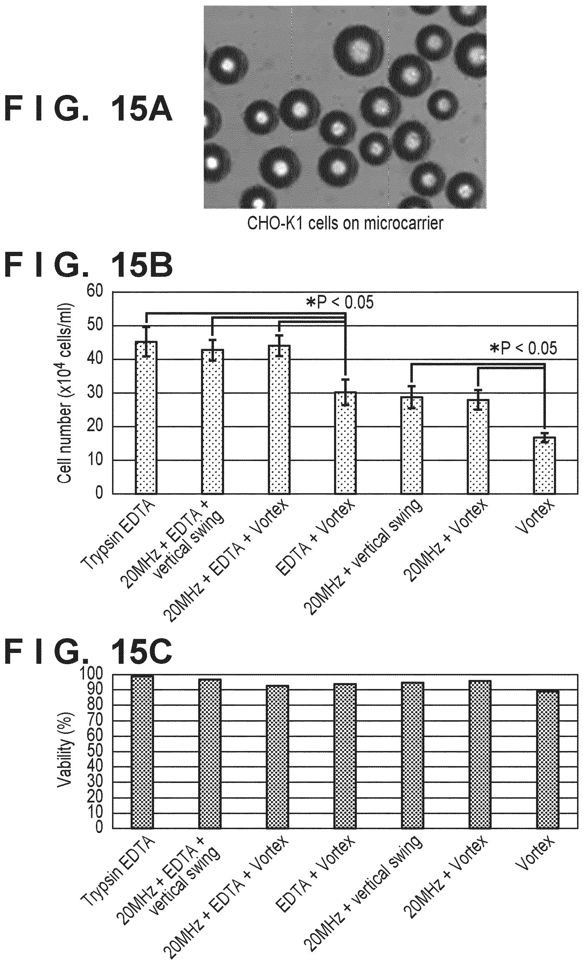

Fig. 15A is a view showing examples of the CHO-K1 cells cultured on the microcarrier beads. The way the CHO-K1 cells were cultured on a plurality of microcarrier beads can be observed.

Fig. 15B shows the measurement results of the number of cells detached under various conditions. In Fig. 15B, each bar marked with "vertical swing" is the result obtained when the alternating field and vertical swing were simultaneously applied, and each bar marked with "vortex" is the result obtained when swing was applied after the application of the alternating field (this similarly applies to Fig. 15C (to be described later)). Note that the vertical swinging process (vertical swing) is 2,500-rpm vertical swing.

Referring to Fig. 15B, the leftmost bar is the result when a trypsin EDTA treatment was performed for 10 min. The second bar from the left is the result when a solution containing only EDTA was added and the vertical swinging process (vertical swing) was performed while applying a ±320-mV/2.6 cm, 20-MHz alternating field. The third bar from the left is the result when a solution containing only EDTA was added, a ±320-mV/2.6 cm, 20-MHz alternating field was applied for 10 min, and then the vortex treatment was performed for 1 min. The fourth bar from the left is the result when the EDTA treatment was performed for 10 min. These bars demonstrate that the application of the alternating field can implement cell detachment equal to that of the trypsin EDTA treatment without using trypsin. The bars also show that the application of the present invention detaches many cells compared to the EDTA treatment.

The third bar from the right in Fig. 15B is the result when a solution not containing EDTA was added and the vertical swinging process (vertical swing) was performed while applying a ±320-mV/2.6 cm, 20-MHz alternating field for 10 min. The second bar from the right in Fig. 15B is the result when a solution not containing EDTA was added, a ±320-mV/2.6 cm, 20-MHz alternating field was applied for 10 min, and then the vortex treatment was performed for 1 min. These bars and the fourth bar from the left reveal that the application of the alternating field can implement cell detachment equal to that of the EDTA treatment without using EDTA. Note that the rightmost bar is the result when a solution not containing EDTA was added and the vortex treatment was performed without applying any alternating field. Thus, it was confirmed that more cells were detached when the alternating field was applied. That is, it was found that even the cells cultured on the microcarrier beads changed the behavior (accelerated detachment) by receiving stimulation by the application of the alternating field.

Fig. 15C shows the measurement results of the viability of cells detached under various conditions. When the trypsin EDTA treatment was performed, the viability was 99% (the leftmost bar in Fig. 15C). When the vertical swinging process (vertical swing) was performed while applying a ±320-mV/2.6 cm, 20-MHz alternating field, the viability was 95% (the third bar from the right in Fig. 15C).

When the ±320-mV/2.6 cm, 20-MHz alternating field was applied for 10 min and then the vortex treatment was performed for 1 min, the viability was 96% (the second bar from the right in Fig. 15C). The viability was about 90% or more under other conditions as well.

From the foregoing, it was found that not only cells cultured on a cell substrate but also cells cultured on microcarrier beads are stimulated and detached by the application of an alternating field.

According to the above-described embodiments explained above, it was confirmed that various effects (for example, the increase in detachment of cells, the increase in viability, and the suppression of the growth of cells) can be given to cells by stimulating the cells by the application of an alternating field.

Note that the configuration in which the electrodes are included in the cell stimulating apparatus is explained in each embodiment described above, but the electrodes need not be included in the cell stimulating apparatus. For example, the upper portion or the bottom portion of the T-25 culture flask 40 or the like may also be formed by an electrode plate and connected to the function generator 10. It is also possible to install a stainless-steel shield around the cell stimulating apparatus in which cells are placed in accordance with the strength of an alternating field, thereby reducing the influence on the periphery of the apparatus.

### [Ninth Embodiment]

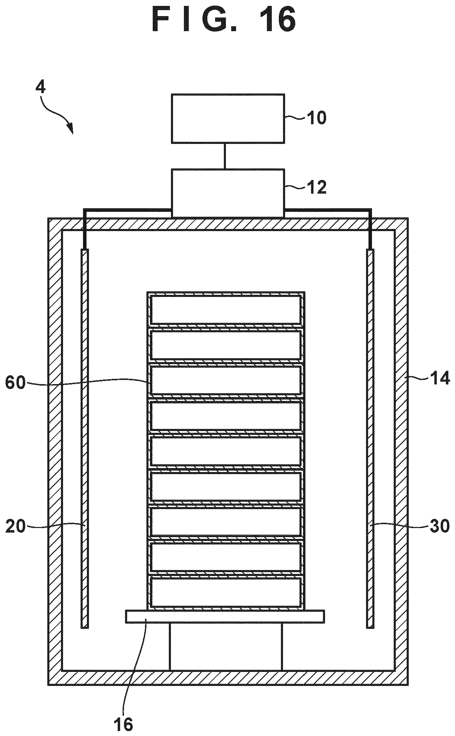

In the ninth embodiment, another form of the cell stimulating apparatus will be explained. A configuration example of a cell stimulating apparatus 4 according to an embodiment of the present invention will be explained below with reference to Fig. 16.

The cell stimulating apparatus 4 is configured as an apparatus that stimulates cells by placing the cells in an alternating field generated between electrodes 20 and 30.

The electrodes 20 and 30 are connected to an amplifier 12. The amplifier 12 receives an electric signal from a function generator 10, amplifies the intensity, and applies the signal to the electrodes. The strength of a field to be generated between the electrodes 20 and 30 can easily be adjusted by using the amplifier 12.

The electrodes 20 and 30 are arranged in a shield 14. The shield 14 confines electromagnetic waves generated inside and does not leak the electromagnetic waves to the outside. Even when the output of the amplifier 12 is raised, therefore, it is possible to prevent the electromagnetic waves (radio waves) generated between the electrodes 20 and 30 from influencing the surroundings.

The cell stimulating apparatus 4 has a support member 16. The support member 16 supports a vessel. The support member 16 is placed inside the shield 14. The support member 16 is preferably made of a material that does not influence the alternating field generated by the electrodes 20 and 30. For example, the support member 16 can be made of a resin material not containing a metal or a magnetic substance.

The support member 16 is so configured as to be able to shake a vessel. For example, the support member 16 can be so configured as to reciprocate or turn horizontally. Alternatively, the support member 16 can be so configured as to move vertically. That is, the support member 16 can be so configured as to be connected to an actuator (not shown) and perform desired motion. Note that in this case, a member of the actuator, which is placed inside the shield 14, is preferably made of a material having no influence on the alternating field.

The support member 16 may also be so configured as to be able to shake a vessel while applying an alternating field to the electrodes 20 and 30. Alternatively, the support member 16 can be so configured as to be able to shake a vessel after applying an alternating field to the electrodes 20 and 30.

A vessel usable in the cell stimulating apparatus 4 is not particularly limited. As shown in Fig. 16, however, it is possible to use a form in which a plurality of vessels are stacked vertically (a so-called stack plate 60). This makes it possible to process a large number of cells at once.

In the cell stimulating apparatus 4, the electrodes 20 and 30 are arranged on the sides of a vessel, and apply an alternating field sideways to the vessel. An alternating field can act on cells in this configuration as well, so the same effect as that of the cell stimulating apparatus 1 can be achieved.

### [10th Embodiment]

In the 10th embodiment, an example in which an alternating field was applied while applying swing will be explained. More specifically, the state of cell detachment was examined when 1200-rpm horizontal turn was performed while vertically applying a ±320-mV/2.6 cm, 20-MHz alternating field to a T-25 culture flask 40 of BALB/3T3 cells in which the depth was set to 2 mm by PBS(-) (condition a).

For comparison, the state of cell detachment when the alternating field was applied without performing the horizontal turning process (condition b) was examined, and the state of cell detachment when only a 1,200-rpm horizontal turning process was performed without applying the alternating field (condition c) was examined.

Consequently, it was found that the time required for cell detachment is shortened by applying an alternating field while giving swing by horizontal turn.

### (Example 10)

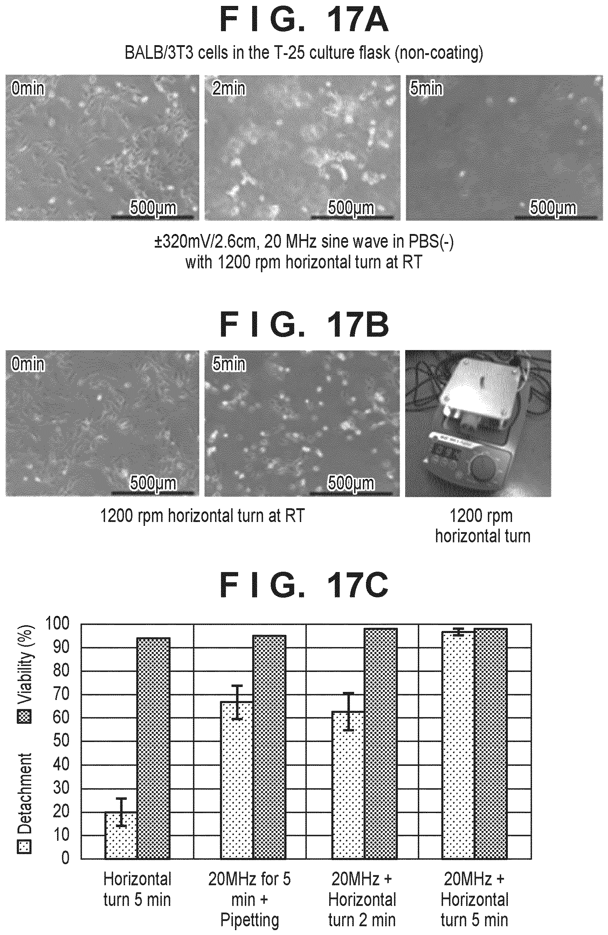

Fig. 17A is a view showing the way cells were detached with time under condition a. Fig. 17A shows states at the times of 0, 2, and 5 min from the left. As a consequence, the cell detachment was 63% for 2 min and 97% for 5 min, as indicated by the second bar from the right and the rightmost bar in Fig. 17C. The cell viability was 98% for both 2 and 5 min.

Fig. 17B is a view showing the way cells were detached with time under condition c. Fig. 17B shows states at the times of 0 and 5 min from the left. The rightmost bar in Fig. 17B shows a state in which the T-25 culture flask 40 sandwiched between the electrodes 20 and 30 was placed on a turning apparatus for performing horizontal turn. Consequently, as indicated by the leftmost bar in Fig. 17C, the cell detachment was 20% for 5 min, and the cell viability was 94% for 5 min.

Also, the second bar from the left in Fig. 17C corresponds to condition b. The cell detachment was about 67% for 5 min, and the cell viability was 95% for 5 min.

From the foregoing, in particular, from the comparison between conditions a and b, it was confirmed that cells were detached faster when horizontal turn was performed while applying the alternating field, than when only the alternating field was applied and no horizontal turn was performed. That is, it was found that the time required for cell detachment is shortened by applying an alternating field while giving swing by horizontal turn.

In addition, when only horizontal turn was performed as in condition c, only 20% of cells was detached at the time of 5 min. As described above, the cell detachment was 67% at the time of 5 min when only the alternating field was applied and no horizontal turn was performed as in condition b. When these values are simply added up, the sum is 87%. When horizontal turn was performed while applying the alternating field as in condition a, the cell detachment was 97% at the time of 5 min, that is, this value is larger. This shows that cell detachment was efficiently performed by combining horizontal turn and the application of the alternating field.

As explained in each of the above-described embodiments, the present invention can easily stimulate cells with a simple configuration.

The present invention is not limited to the above embodiments and various changes and modifications can be made within the spirit and scope of the present invention. Therefore, to apprise the public of the scope of the present invention, the following claims are made.

This application claims priority from Japanese Patent Application No. 2018-139593 filed July 25, 2018, which is hereby incorporated by reference herein.

## Claims

1. A cell stimulating apparatus for detaching adhesive cells adhered on a culture substrate from the culture substrate by stimulating the cells, **characterized by** comprising:
application means for applying an alternating field between a first electrode and a second electrode opposing each other by sandwiching cells and a solution in which the cells are dipped,
wherein at least one of the first electrode and the second electrode is not in contact with the cells and the solution.

2. The cell stimulating apparatus according to claim 1, **characterized in that** the first electrode and the second electrode are parallel plate electrodes.

3. The cell stimulating apparatus according to claim 2, **characterized in that** the first electrode and the second electrode vertically sandwich the cells.

4. The cell stimulating apparatus according to claim 2, **characterized in that** the first electrode and the second electrode horizontally sandwich the cells.

5. The cell stimulating apparatus according to any one of claims 1 to 4, **characterized in that** a frequency of the alternating field is 0.5 to 1,000 MHz.

6. The cell stimulating apparatus according to any one of claims 1 to 5, **characterized in that** a strength of the alternating field is not less than ±0.3 V/m.

7. The cell stimulating apparatus according to any one of claims 1 to 6, **characterized in that**
the cells and the solution are contained in a vessel, and
the first electrode and the second electrode are arranged outside the vessel.

8. The cell stimulating apparatus according to any one of claims 1 to 7, **characterized in that**
the cells and the solution are contained in a vessel, and
the first electrode and the second electrode oppose each other by sandwiching a plurality of stacked vessels.

9. The cell stimulating apparatus according to any one of claims 1 to 7, **characterized in that**
the cells and the solution are contained in a vessel, and
the first electrode and the second electrode oppose each other by sandwiching a plurality of vessels arranged parallel to each other.

10. The cell stimulating apparatus according to any one of claims 1 to 9, **characterized in that**
the cells and the solution are contained in a vessel, and
the apparatus further comprises shaking means for shaking the vessel.

11. The cell stimulating apparatus according to claim 10, **characterized in that** the shaking means shakes the vessel while applying the alternating field.

12. The cell stimulating apparatus according to claim 10, **characterized in that** the shaking means shakes the vessel after applying the alternating field.

13. The cell stimulating apparatus according to any one of claims 1 to 12, **characterized by** further comprising shielding means surrounding the first electrode and the second electrode.

14. The cell stimulating apparatus according to any one of claims 1 to 13, **characterized by** further comprising measurement means for measuring a voltage between the first electrode and the second electrode.

15. The cell stimulating apparatus according to claim 14, **characterized in that** the application means adjusts an output based on a measurement value of the measurement means.

16. The cell stimulating apparatus according to any one of claims 1 to 15, **characterized in that** the culture substrate is a plate-like culture substrate.

17. The cell stimulating apparatus according to any one of claims 1 to 16, **characterized in that** the culture substrate is a microcarrier.

18. A cell stimulating method for detaching adhesive cells adhered on a culture substrate from the culture substrate by stimulating the cells, **characterized by** comprising:
a step of placing cells and a solution in which the cells are dipped, between a first electrode and a second electrode opposing the first electrode; and
a step of applying an alternating field between the first electrode and the second electrode,
wherein at least one of the first electrode and the second electrode is not in contact with the cells and the solution.

19. A culture product manufacturing method including a cell stimulating method cited in claim 18.

20. An isolated cell manufacturing method **characterized by** comprising:
a step of placing adhesive cells adhered on a culture substrate and a solution in which the cells are dipped, between a first electrode and a second electrode opposing the first electrode; and
a step of applying an alternating field between the first electrode and the second electrode such that the cells are detached from the culture substrate and isolated,
wherein at least one of the first electrode and the second electrode is not in contact with the cells and the solution.

21. A cell growing method **characterized by** comprising:
a step of placing adhesive cells adhered on a culture substrate and a solution in which the cells are dipped, between a first electrode and a second electrode opposing the first electrode;
a step of applying an alternating field between the first electrode and the second electrode such that the cells are detached from the culture substrate and isolated; and
a step of culturing and growing the isolated cells detached from the culture substrate,
wherein at least one of the first electrode and the second electrode is not in contact with the cells and the solution.
